(19)

Europäisches
Patentamt
European
Patent Office
Office européen
des brevets

(11)     **EP 2 664 319 A1**

(12)     **EUROPEAN PATENT APPLICATION**
published in accordance with Art. 153(4) EPC

(43) Date of publication:
**20.11.2013 Bulletin 2013/47**

(21) Application number: **12733838.2**

(22) Date of filing: **11.01.2012**

(51) Int Cl.:
*A61K 8/36* (2006.01)          *A61K 8/19* (2006.01)
*A61K 8/86* (2006.01)          *A61Q 19/10* (2006.01)

(86) International application number:
**PCT/JP2012/050327**

(87) International publication number:
**WO 2012/096278 (19.07.2012 Gazette 2012/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.01.2011 JP 2011003624**

(71) Applicant: **Kao Corporation
Chuo-Ku
Tokyo 103-8210 (JP)**

(72) Inventors:
• **NONAKA Nobuhiro
Wakayama-shi
Wakayama 640-8580 (JP)**

• **IMAIZUMI Yoshinobu
Wakayama-shi
Wakayama 640-8580 (JP)**
• **TANABE Yuichi
Tokyo 131-8501 (JP)**

(74) Representative: **HOFFMANN EITLE
Patent- und Rechtsanwälte
Arabellastrasse 4
81925 München (DE)**

(54)     **PROCESS FOR PRODUCTION OF EFFERVESCENT BATH AGENT COMPOSITION**

(57)     The present invention discloses a method for producing an effervescent bath agent composition, including the following steps:
step 1: mixing an organic acid, not less than 0.1 parts by mass, to 100 parts by mass of the organic acid, of a nonionic surfactant and a water-soluble binder to obtain a mixture;
step 2: granulating, compress-molding, or and compress-molding after granulating, the mixture obtained in step 1 to obtain a granulated or compress-molded product; and
step 3 : mixing the granulated or compress-molded product obtained in step 2 with a carbonate.

EP 2 664 319 A1

**Description**

Field of the invention

The present invention relates to a method for producing an effervescent bath agent composition.

Background of the invention

There have been known bath tablets generating carbon dioxide through a reaction of a carbonate and an organic acid (JP-A 59-70609 and JP-B 3230327). The conventional bath agents are insufficient in effects to warm the body in soaking in a bathtub. There has been thus a demand for improved effects to warm the body.
JP-B 3230327 discloses an effervescent bath agent, containing an organic acid, a carbonate, and a binder. This bathing agent, however, generates coarse gas bubbles of carbon dioxide during bubbling and is insufficient to satisfy bubbling performance of carbon dioxide.
Under such circumstances, there has been a demand for an effervescent bath agent composition having sufficient effects to warm the body and well generating gas bubbles of carbon dioxide.
JP-A 2011-021005, published on Feb. 3, 2011, discloses a powdered effervescent bath agent composition, containing granules including an organic acid, an oil component, a nonionic surfactant, and a water-soluble binder, and a carbonate.
JP-A2011-021004, published on Feb. 3, 2011, discloses a method for producing a powdered effervescent bath agent composition, using granules containing an organic acid, an oil component, a nonionic surfactant, and a water-soluble binder, and a carbonate.
JP-B 05-069805 discloses a bath agent containing an organic acid and a carbonate.

Summary of the invention

The present invention provides a method for producing an effervescent bath agent composition, including the following steps:

step 1: mixing an organic acid, a nonionic surfactant in the amount of not less than 0.1 part by mass, to 100 parts by mass of the organic acid, and a water-soluble binder to obtain a mixture;
step 2: granulating, or compress-molding, or granulating and then compress-molding, the mixture obtained in step 1 to obtain a granulated or compress-molded product; and
step 3 : mixing the granulated or compress-molded product obtained in step 2 with a carbonate.

Detailed description of the invention

The present invention provides a method for producing an effervescent bath agent composition having sufficient effects to warm the body and well generating gas bubbles of carbon dioxide.
The present inventors have found that an effervescent bath agent composition, produced by mixing an organic acid, a nonionic surfactant at a specific amount ratio to the organic  acid, and a water-soluble binder to obtain a mixture, granulating, compress-molding, or granulating and compress-molding the mixture to form a granulated or compress-molded product, and mixing the granulated or compress-molded product with a carbonate, generates very fine gas bubbles of carbon dioxide in bathwater, or an effervescent bath agent composition having good effects to warm the body is reduced to practice this way, accomplished the present invention.
According to the method of the present invention, provided is an effervescent bath agent composition that can generate very fine gas bubbles of carbon dioxide in bathwater to dissolve an increased amount of carbon dioxide in the bathwater or increase a carbon dioxide concentration in the bathwater, thereby achieving good effects to promote blood circulation.
The generation of fine bubbles makes the bathwater cloudy through a bathtub. The appearance of the bathwater provides a feeling of sufficient bubbling to build a comfortable feeling of greater bath time.
An effervescent bath agent composition, produced by the method of the present invention, includes a granulated or compress-molded product, prepared by mixing an organic acid, not less than 0.1 parts by mass of nonionic surfactant, to organic acid 100 parts by mass, and a water-soluble binder to obtain a mixture, granulating, compress-molding, or granulating and compress-molding the mixture, and a carbonate. The bath agent composition includes a solid matter formed by granulating or compress-molding. The solid matter may be in a form of tablet, briquette, powder, or the like.

[Organic acid]

The organic acid used in the present invention is a component for neutralizing a carbonate to generate carbon dioxide.

For carrying a liquid nonionic surfactant, the organic acid preferably has an oil absorption capacity of not less than 0.02 ml/g, and more preferably 0.05 ml/g. The oil absorption capacity is a value determined by the method described in Examples. Although the upper limit of the oil absorption capacity is not specifically limited, the organic acid preferably has an oil absorption capacity of not more than 1.0 ml/g.

From the viewpoints of solubility and easiness in handling, the organic acid is preferably water-soluble and in the solid state.

Examples of the organic acid preferably used include succinic acid, fumaric acid, malic acid, adipic acid, tartaric acid, benzoic acid, citric acid, pyrrolidonecarboxylic acid, and salicylic acid. The organic acid is, among others, preferably at least one acid selected from fumaric acid, succinic acid, malic acid and citric acid. From the viewpoints of easiness in handling, a low hygroscopicity and economic efficiency, more preferable are succinic acid and fumaric acid. These organic acids may be used alone or optionally in combination of two or more of them.

From the viewpoints of granulation, solubility, and oil absorption capacity, the organic acid preferably has a mean particle diameter of not more than 250 $\mu$m, more preferably not more than 200 $\mu$m, and even more preferably not more than 150 $\mu$m. For achieving higher satisfaction with granulation, solubility, and oil absorption capacity, the mean particle diameter is particularly preferably not more than 100 $\mu$m, and more preferably not more than 50 $\mu$m. From the viewpoint of productivity, the lower limit of the mean particle diameter is preferably not less than 10 $\mu$m.

Larger granules than the aforementioned diameter are preferably crushed into an appropriate size with a crushing means. Examples of the crushing means include mechanical impact crushers such as a hammer crusher, mechanical impact pulverizers such as an atomizer and a pin mill, and shearing crushers such as a flush mill. Larger granules may be crushed in a one step or in multiple steps with a kind of crusher or different crushers.

A content of the organic acid in the bath agent composition is preferably 30 to 80% by mass, more preferably 40 to 70% by mass, and even more preferably 40 to 60% by mass. The lower limit, above shown, is preferable for the organic acid to carry a liquid component stably and to maintain the shape retention of granules.

[Nonionic surfactant]

The effervescent bath agent composition of the present invention includes the nonionic surfactant in an amount of not less than 0.1 parts by mass to 100 parts by mass of organic acid. For increasing dispersibility/solubility of the organic acid and generating fine gas bubbles of carbon dioxide, the amount is preferably not less than 1 part by mass, more preferably not less than 3 parts by mass, and even more preferably not less than 5 parts by mass to 100 parts by mass of organic acid. The upper limit of the amount is not specifically limited. However, for preventing powder from caking or a liquid component from bleeding out during compress-molding, the amount is preferably not more than 20 parts by mass, and more preferably not more than 15 parts by mass to 100 parts by mass of organic acid. Collecting them together, the amount is preferably 0.1 to 20 parts by mass, more preferably 1 to 20 parts by mass, even more preferably 1 to 15 parts by mass, still even more preferably 3 to 15 parts by mass, and yet still even more preferably 5 to 15 parts by mass to 100 parts by mass of organic acid.

Examples of the nonionic surfactant include glycerol fatty acid esters, propylene glycol fatty acid esters, sorbitan fatty acid esters, polyoxyethylene sorbitan fatty acid esters, sucrose fatty acid esters, tetraoleic acid polyoxyethylene sorbits, polyoxyethylene alkyl ethers, polyoxyethylene alkenyl ethers, polyoxyethylene polyoxypropylene glycols, polyoxyethylene polyoxypropylene alkyl ethers, polyoxyethylene polyoxypropylene alkenyl ethers, polyethylene glycol fatty acid esters, polyoxyethylene castor oils, polyoxyethylene hydrogenated castor oils, and polyglycerol fatty acid esters. For generating fine gas bubbles of carbon dioxide, preferred are fatty acid esters having a polyoxyalkylene group, and more preferred are polyoxyethylene sorbitan fatty acid esters.

These nonionic surfactants may be used alone or optionally in combination of two or more of them.

The nonionic surfactant may be in a liquid, powder, paste, or wax form at an ambient temperature (25°C). For increasing dispersibility/solubility of the nonionic surfactant in bathwater, the nonionic surfactant preferably has a melting point of not higher than 40°C and is in the form of liquid or paste, and more preferably in a liquid form at an ambient temperature (25°C).

For generating fine gas bubbles of carbon dioxide, the nonionic surfactant preferably has HLB of 7 to 20, more preferably 7.5 to 20, even more preferably 8 to 20, still even more preferably 8 to 18, and yet still even more preferably 8 to 15.

As used herein, the "HLB" is a value commonly used for expressing a balance between hydrophilic property and hydrophobic property of a surfactant. It can be determined by several calculation methods, including Kawakami's method, used commonly in the art. The present invention employs the Kawakami's method as shown below:

$$HLB = 7 + 11.7 \log_{10} (M_w/M_0)$$

wherein,

$M_w$ is the total molecular mass of a hydrophilic portion; and

$M_0$ is the total molecular mass of a lipophilic portion.

From the viewpoints of amount of carbon dioxide generating and size of a gas bubble, a content of the nonionic surfactant in the bath agent composition is preferably 0.5 to 12% by mass, more preferably 1 to 10% by mass, and even more preferably 2 to 8% by mass.

[Water-soluble binder]

The water-soluble binder used in the present invention is a component for increasing strength of a granulated or compress-molded product. The organic acid is coated with the water-soluble binder to prevent the organic acid and the  carbonate from being in contact with each other and reacting each other in a package during storage and then preventing carbon dioxide from generating and prevent the package from expanding.

From the viewpoint of granulation, the water-soluble binder is preferably thermoplastic. The water-soluble binder may be in the form of an aqueous solution or not. The aqueous solution of the water-soluble binder should be dried to be separated from water before granulating in order to suppress the reaction between the organic acid and the carbonate. From the viewpoints of production cost and productivity, the water-soluble binder is preferably not in the form of aqueous solution.

Examples of such a water-soluble binder include polyoxyalkylene glycols such as polyethylene glycol and polypropylene glycol. Polyethylene glycol and polypropylene glycol are particularly preferred.

From the viewpoints of viscosity control and easiness in handling in granulating or compress-molding, the water-soluble binder preferably has a number average-molecular weight of 4,000 to 20,000, more preferably 6,000 to 13,000, and even more preferably 7,000 to 9,000 as measured by GPC based on a polystyrene standard. In cases of using polyethylene glycol as the water-soluble binder, a number average-molecular weight thereof was measured using water/ethanol as a solvent.

The water-soluble binder may be a mixture of two or more binders having different number-average-molecular weights.

From the viewpoint of particle strength, a content of  the water-soluble binder in the bath agent composition is preferably 2 to 20% by mass, and more preferably 5 to 15% by mass. With the content equal to or higher than the lower limit, the produced granule has increased particle strength and hardly breaks during transportation in production. With the content equal to or lower than the upper limit, the bath agent composition has increased solubility in bathwater and generates more bubbles.

[Carbonate]

The carbonate used in the present invention is a component for generating carbon dioxide through neutralization by reacting with the organic acid.

For producing finer gas bubbles of carbon dioxide, the carbonate is preferably selected from sodium carbonate, potassium carbonate, and sodium hydrogen carbonate, and more preferably sodium carbonate ($Na_2CO_3$).

From the viewpoints of amount and fineness of gas bubbles of carbon dioxide generating, a content of the carbonate in the bath agent composition is preferably 20 to 70% by mass, more preferably 30 to 60% by mass, and even more preferably 30 to 50% by mass.

For producing fine gas bubbles, a content of sodium carbonate in the carbonate is preferably 70 to 100% by mass, more preferably 80 to 100% by mass, and even more preferably 85 to 100% by mass.

Amass ratio of the organic acid to the carbonate, organic acid/carbonate, is preferably 30/70 to 80/20, more preferably 40/60 to 70/30, and even more preferably 40/60 to 60/40.

From the viewpoints of fineness, stability of bubbles, and sedimentation, the carbonate preferably has a mean particle size of 100 to 750 $\mu$m, more preferably 200 to 500 $\mu$m, and even more preferably 250 to 400 $\mu$m.

[Other components]

The bath agent composition of the present invention can further contain an optional component other than those described above within the range that the purpose of the present invention can be achieved.

[Oil component]

The bath agent composition of the present invention may further contain an oil component. Carbon dioxide generated from the bath agent composition in bathwater becomes distributed not only into the bathwater, but the oil component. Accordingly, a higher concentration of carbon dioxide is delivered to the skin to provide a stronger feel of having warmed after taking a bath.

Although the oil component is effective for enhancing a feeling of having warmed after taking a bath, it undesirably tends to decrease dispersibility/solubility of the organic acid in water and generation of fine bubbles of carbon dioxide. Further, a granulated or compress-molded product containing the oil component may have decreased particle strength. Considering these properties and for generating fine bubbles of carbon dioxide and achieving sufficient particle strength, the oil component is preferably added in a specific amount. The amount of the oil component is preferably 0 to 4 parts by mass, more preferably 0 to 3 parts by mass, more preferably 0 to 2 parts by mass, more preferably 0 to 1 parts by mass, more preferably 0 to 0.5 parts by mass, more preferably 0 to 0.1 parts by mass to 100 parts by mass of organic acid. The oil component is even more preferably not contained, or 0 part by mass.

The oil component used preferably provides partition of carbon dioxide into an oil phase and an aqueous phase at a ratio, oil phase/aqueous phase, of not less than 1.1 to enhance a feeling of having warmed after taking a bath. The distribution ratio is preferably not less than 1.3, more preferably not less than 1.5, even more preferably not less than 1.6, and still even more preferably not less than 1.7.

Examples of the oil component include: fatty acid esters such as cetyl octanoate, cetyl isooctanoate, myristyl lactate, cetyl lactate, isopropyl myristate, myristyl myristate, isocetyl myristate, octyldodecyl myristate, isopropyl palmitate, isostearyl palmitate, isopropyl adipate, butyl stearate, isocetyl stearate, isostearyl isostearate, isotridecyl isononanoate, decyl oleate, and cholesterol isostearate;

fatty acid triglycerides such as glycerol tri-(caprylate-caprate) and glyceryl tricaprylate;

natural fats and oils such as soy oil, rice bran oil, jojoba oil, avocado oil, almond oil, olive oil, cocoa butter, sesame oil, persic oil, castor oil, palm oil, mink oil, tallow, and lard, hardened oil obtained by hydrogenating the natural fats and oils, and glycerides such as myristic acid glyceride and 2-ethylhexanoic acid glyceride;

hydrocarbon oils such as liquid paraffin, Vaseline, paraffin, microcrystalline wax, ceresin, squalane, squalene, dioctylcyclohexane, and pristane;

higher fatty acids such as lauric acid, myristic acid, palmitic acid, stearic acid, behenic acid, oleic acid, linoleic acid, linolenic acid, lanolic acid, and isostearic acid;

higher alcohols such as lauryl alcohol, cetyl alcohol, stearyl alcohol, oleyl alcohol, cholesterol, and 2-hexyldecanol;

essential oils such as mint oil, jasmine oil, camphor oil, cypress oil, spruce oil, ryu-yu, turpentine oil, cinnamon oil, bergamot oil, orange oil, calamus oil, pine oil, lavender oil, bay oil, clove oil, Hiva (Thujopsis dolabrata) oil, rose oil, eucalyptus oil, lemon oil, thyme oil, peppermint oil, rose oil, sage oil, menthol, cineol, eugenol, citral, citronellal, borneol, linalool, geraniol, camphor, thymol, spilanthol, pinene, limonene, and terpenes; and

silicone oils.

When two kinds of oil components are contained, the above shown distribution ratio is a ratio in a mixture thereof. Even an oil component that provides the distribution ratio of lower than 1.1 in itself can provide the distribution ratio of not lower than 1.1 when it is combined with another oil component. The distribution ratio of carbon dioxide between an oil phase/an aqueous phase is measured by a method described in JP-A 2005-314233, paragraph 26. A distribution ratio of carbon dioxide between an oil phase and an aqueous phase is determined by the method described in Examples.

Examples of the oil component that can provide the distribution ratio of not lower than 1.7 by itself include jojoba oil, squalene, isopropyl myristate, isocetyl myristate, isopropyl palmitate, isocetyl stearate, cetyl isooctanoate, isotridecyl isononanoate, dioctylcyclohexane, glycerol tri-(caprylate-caprate), and glyceryl tricaprylate.

The bath agent composition preferably contains an oil component that can provide the distribution ratio of not lower than 1.7 by itself, selected from those described above, and it more preferably has the distribution ratio of not less than 1.1.

In cases of formulating the oil component, for homogeneously emulsifying the oil component in bathwater, the oil component is preferably combined with at least one nonionic surfactant selected from polyoxyethylene alkyl ethers and polyoxyethylene alkenyl ethers and at least one nonionic surfactant selected from tetraoleic acid polyoxyethylene sorbit and polyoxyethylene sorbitan fatty acid esters.

[Anion surfactant]

Even when an oil component is contained, the dispersibility/solubility of the organic acid in water is improved by further containing an anion surfactant in addition to the nonionic surfactants. Even when an oil component is contained, generation of fine gas bubbles of carbon dioxide is enhanced.

Accordingly, it is also a preferred embodiment to further add the oil component and the anionic surfactant in step 1.

Examples of the anion surfactant include sodium alkylsulfates, polyoxyethylene alkyl ether sulfuric acid sodium salts, polyoxyethylene alkyl ether acetic acid sodium salts, fatty acid soaps, monoalkylphosphate salts, N-acylglutamic acid salts, and N-acylmethyltaurine salts, of which an alkyl group preferably has 8 to 20 carbon atoms, and more preferably 8 to 14 atoms.

Among these anion surfactants, preferred are polyoxyethylene alkyl ether acetic acid sodium salts having 1 to 20 mole number of polyoxyethylene groups on the average, because they increase the dispersibility/solubility of the organic acid in water even in a small amount.

An amount of the anion surfactant added is preferably 0.1 to 3 parts by mass, more preferably 0.2 to 2 parts by mass, and even more preferably 0.3 to 1 parts by mass to 100 parts by mass of organic acid.

In cases of using the anion surfactant, the oil component is preferably used in an amount of 0.3 to 4 parts by mass, more preferably 0.5 to 3 parts by mass, and even more preferably 1 to 3 parts by mass to 100 parts by mass of organic acid. An amount of the anion surfactant is preferably 5 to 60 parts by mass, and more preferably 10 to 40 parts by mass to 100 parts by mass of oil component.

[Disintegrating agent]

The bath agent composition can further contain a disintegrating agent. The disintegrating agent facilitates disintegration of a granulated or compress-molded product to increase the disintegration and the solubility in bathwater. The disintegrating agent is preferably selected from saccharides and inorganic acids.

Examples of the sugar include glucose, fructose, lactose, maltose, sucrose, dextrin, maltodextrin, cyclodextrin, maltose, fructose, and trehalose.

Examples of the inorganic acid include sodium sulfate, magnesium sulfate, sodium chloride, potassium chloride, magnesium oxide, sodium polyphosphate, and sodium phosphate.

From the viewpoint of disintegration effect, the disintegrating agent preferably has a mean particle size of not more than 300 $\mu$m, more preferably not more than 200 $\mu$m, and even more preferably not more than 100 $\mu$m. In granulation by extrusion, the disintegrating agent used preferably has a mean particle size of not more than 50 $\mu$m, and more preferably not more than 30 $\mu$m.

For increasing solubility in bathwater, a content of the disintegrating agent in the bath agent composition is preferably not less than 0.1% by mass, and more preferably not less than 1% by mass. From the cost viewpoint, the content is preferably not more than 10% by mass, and more preferably not more than 5% by mass for the upper limit. Collecting them together, the content of the disintegrating agent in the bath agent composition is preferably 0.01 to 10% by mass, more preferably 0.1 to 10% by mass, and even more preferably 1 to 6% by mass.

[Antioxidant]

In cases of using a compound having an unsaturated carbon bond as the oil component or the nonionic surfactant, for preventing scent from changing due to oxidation under temperature conditions during production and storage of the bath agent composition, the bath agent composition preferably further contains an antioxidant such as rutin, polyphenols such as proanthocyanidin and catechin, vitamin C and tocopherol.

For controlling a change in scent and retarding reduction of generation of fine bubbles of carbon dioxide after a long-term storage, a content of the antioxidant in the bath agent composition is preferably 0.01 to 5% by mass, more preferably 0.02 to 1% by mass, and even more preferably 0.03 to 0.1% by mass.

The bath agent composition can further contain other auxiliary agent such as a perfume, a bactericide/fungicide, and a colorant.

The bath agent composition can further contain a component commonly used in bath agents within the range that does not impair the effects of the present invention. Examples of the component include, but not limited to, inorganic salts thereof such as disintegrating agents such as saccharides and inorganic acid as described above, sodium chloride, sodium sulfate, potassium nitrate, sodium nitrate, calcium nitrate, sodium polyphosphate, ammonium chloride, iron sulfate, sodium phosphate, sodium thiosulfate, boric acid, metasilicic acid, and anhydrous silicic acid;

herbal medicines such as Atractylodes lancea rhizome, white Atractylodes rhizome, Valeriana fauriei rhizome/root, Schizonepeta tenuifolia, magnolia bark, Cnidium rhizome, orange peel, Japanese Angelica root, ginger powder, Ginseng, Cinnamon bark, Peony root, mint leaf, Scutellaria root, Gardenia fruit, Poria sclerotium, Aralia rhizome, Acorus calamus rhizome, gaiyou, Schisandra vine, Angelica dahurica root, Houttuynia herb, Dryobalanops aromatica extract, saffron, Phellodendron bark extract, Citrus unshiu peel, fennel, Citrus unshiu peel powder, chamomile, melissa (lemon balm), rosemary, horse chestnut, yarrow (Achillea millefolium), and arnica;

alcohols such as ethanol and isopropyl alcohol;

polyhydric alcohols such as glycerol, propylene glycol, and sorbitol;

and other components in production such as titanium oxide, zinc oxide, talc, sulfur, mineral sand, yunohana (deposits from hot springs), casein, neutral clay, sodium salicylate, sodium carboxymethylcellulose, yolk powder, roasted rice bran, mica powder, skim milk powder, an antimicrobial preservative, a moisturizing agent, a sequestering agent, a perfume, a colorant.

<Method for producing an effervescent bath agent composition

The method for producing an effervescent bath agent composition of the present invention includes the following steps:

step 1: mixing 100 parts by mass of an organic acid, not less than 0.1 parts by mass of a nonionic surfactant, and a water-soluble binder to obtain a mixture;
step 2: granulating, compress-molding, or granulating and compress-molding the mixture obtained in step 1 to form a granulated or compress-molded product; and
step 3: mixing the granulated or compress-molded product obtained in step 2 with a carbonate.

A part of the carbonate may be added in step 1 and/or step 2 insofar as the effects of the present invention is achieved.

[Step 1]

Step 1 is to mix 100 parts by mass of an organic acid, not less than 0.1 part by mass of a nonionic surfactant and a water-soluble binder to obtain a mixture.
Step 1 is preferably conducted by first mixing 100 parts by mass of an organic acid and not less than 0.1 part by mass of a nonionic surfactant and then adding a water-soluble binder and mixing them.
The water-soluble binder may be partially or fully molten in advance to be applied or applied in a solid state and molten by heating in a mixer. The water-soluble binder is preferably applied in a molten state in advance.
In cases of adding the oil component in step 1, for generating fine gas bubbles of carbon dioxide in bathwater, step 1 is preferably conducted by first mixing the organic acid and at least a part of not less than 0.1 part by mass of the nonionic surfactant to 100 parts by mass of the organic acid, and then mixing with the water-soluble binder, the rest of the nonionic surfactant, and the oil component.
In step 1, any apparatus can be used for mixing components, provided that the apparatus does not apply so strong a shearing force as causing a heavy consolidation. Examples of the apparatus include a drum mixer, a ribbon mixer, and a Nauta mixer.
Even mixers essentially applying high shearing force such as a Schugi mixer, a Henschel mixer, a Loedige mixer having a main impeller and a crushing blade in a horizontal or vertical system, and a high-speed mixer can be used under limited conditions to a small rotation number and a low Froude number described below for controlling consolidation.
For controlling consolidation during granulation, a Froude number, which is defined by the following equation, of the mixing apparatus is preferably set to not more than 20, more preferably not more than 10, even more preferably not more than 5, still even more preferably not more than 3, and yet still even more preferably not more than 2.
$$\text{Froude number: } Fr = V^2/(R \times g)$$

V: circumferential speed [m/s]
R: radius of a circle of a rotating object [m] g: gravitational acceleration [m/s$^2$]
In cases of vertical or horizontal mixing apparatuses having a main impeller and a crushing blade, V and R are values based on a main shaft. In cases of a Nauta mixer having a mixing unit with rotation on its own axis and revolution, V and R are values based on a rotation axis on its own axis.
In step 1, for suppressing the organic acid from subliming, components are preferably mixed at a temperature of not higher than 150°C, more preferably 20°C to 150°C, even more preferably 30°C to 100°C, and still even more preferably 40°C to 80°C.

[Step 2]

Step 2 is to granulate, compress, or granulate and compress the mixture, obtained in step 1, to form a granulated or compress-molded product.

Granulation can be performed with a known extrusion granulator, such as Pereter double (twin-screw extruder), Dome granulator, Twin Dome granulator, Disk pereter (manufactured by Dalton Co. , Ltd.), or a basket granulator (Kikusui Seisakusho Ltd.), or a known tumbling granulator, such as Henschel mixer (manufactured by Nippon Coke & Engineering Co., Ltd.), High-speed mixer (manufactured by Fukae Powtech or Earth Technica Co., Ltd.), Vertical granulator (manufactured by Powrex Corporation), Loedige mixer (manufactured by Matsubo corporation), Ploughshare mixer (manufactured by Pacific Machinery & Engineering Co., Ltd.), V-Shaped mixer (manufactured by Fuji Paudal Co., Ltd.), Ribbon mixer (manufactured by Hosokawa Micron Group), Nauta mixer (manufactured by Hosokawa Micron Group), or SV mixer (manufactured by Shinko Pantech or Kobelco Eco-Solutions Co., Ltd.). The granulation is preferably extrusion granulation performed with an extrusion granulator.

In cases of using an extrusion granulator, the granulator preferably uses a screen with an opening size of 0.3 to 2.0 mm, more preferably 0.5 to 2.0 mm, and even more preferably 0.7 to 1.0 mm. Use of such a screen enables to form a bar or noodle of granules.

The product from extrusion granulation is cooled for suppressing from unifying or aggregation, and if needed, can be subjected to size regulation. In size regulation, a known grinder (or crusher) can be used, including High-speed mixer (manufactured by Earthtechnica Co., Ltd.), Marmerizer (manufactured by Dalton Co., Ltd.), Spir-A-Flow (manufactured by Freund Corporation), Fitz mill (manufactured by Dalton Co. , Ltd.), Power Mill (manufactured by Powrex Corporation), and Comil (manufactured by Quadro Engineering), and the like.

Compression molding can be performed with a briquetting machine, a tableting machine, or a known apparatus that can produce a briquette or a tablet. A briquetting machine includes two rotatable rolls, each roll having briquetting pockets in an outer periphery thereof to serve as a mother die of a desired briquette, the rolls being arranged to be engaged into each other, rotating at the same rate as each other, continuously feeding a material between the rotating rolls to compress the material to form briquettes. As a well-known briquetting machine, a briquetting machine, manufactured by Sintokogio Ltd. etc. can be used. A tableting machine is an apparatus, including dies, with which a material is fed in the die and the material is compressed and molded with the dies. There is a single stroke type containing one die and a pair of dies moving  up and down for compressing. There is a rotary type containing a horizontal turntable having dies equally spaced along the periphery, where the turntable rotates and a continued sequence of operations of filling, compressing and discharging. There are well-known tableting machines such as a single stroke tableting machine manufactured by Riken and a rotary tableting machine manufactured by Kikusui Seisakusho Ltd.

In cases of compress-molding the mixture in step 2, from the viewpoint of easiness in handling such as in feeding to a compression molding machine, the compression is preferably performed after the granulation of the mixture obtained in step 1.

In cases of using a briquetting machine, from the viewpoint of solubility of a briquette, a produced briquette preferably has a particle size of not more than 10 mm, more preferably not more than 7 mm, and even more preferably not more than 5 mm. A produced briquette can be in any form. Examples of a preferred form include a so-called briquette, an almond, a pillow, a finger, and a lens forms.

In cases of using a tableting machine, a produced tablet preferably has a cylindrical diameter (or a length of a polygon side) of not more than 80 mm and a thickness of not more than 20 mm. A produced tablet can be in any form. Examples of a preferred form include a cylindrical and a polygonal forms.

[Step 3]

Step 3 is to mix the granulated or compress-molded product from step 2 with a carbonate. Step 3 can be conducted by any method, provided that the method can provide a substantially uniform mixture of the granulated or compress-molded product obtained in step 2 with the carbonate. For example, mixing may be performed with the mixer described in step 1, or with a V-shaped blender (manufactured by Powrex Corporation), a W cone blender (manufactured by Tokuju Corporation), a Ribbon blender (manufactured by Hosokawa Micron Group), or an SV MIXER (manufactured by Kobelco Eco-Solutions Co., Ltd.), or the like.

[Step 4]

The method of the present invention may further include step 4 of granulating or compress-molding the mixture resulting from step 3 to form a granulated or compress-molded product. For granulating or compress-molding, as described in step 2, an extrusion granulator, a tumbling granulator, a briquetting machine, and a tableting machine can be used.

In step 4, the granulated or compress-molded product resulting from step 3 can be mixed with a part of the water-soluble binder using the mixer shown in step 1 and then granulated or compress-molded.

The bath agent composition of the present invention preferably has pH of 5 to 7, and more preferably 5.5 to 6.5 in an aqueous 0.01% by mass solution at 25°C. At pH of 5 to 7, generated carbon dioxide dissolves in bathwater in a larger amount to enhance effects of promoting blood circulation. In the present invention, the pH is measured with a Horiba pH meter (D-51S).

When dissolving in bathwater, the bath agent composition of the present invention generates fine bubbles in a large amount for a long time. Generation of fine bubbles can be confirmed as fast clouding of bathwater with these bubbles when  the bath agent composition of the present invention in an amount of 30 to 80 g is dissolved in 180 L of bathwater.

When used, the bath agent composition of the present invention is dissolved in bathwater to generate gas bubbles of carbon dioxide. The bath agent composition of the present invention can also be used in partial soaking such as foot and arm baths, as well as full soaking in taking a bath.

The bath agent composition of the present invention has good fluidity and can be put in a pillow or bottle to be used.

The present invention includes the following preferred aspects:

[1] A method for producing an effervescent bath agent composition, including the following steps:

step 1: mixing an organic acid, a nonionic surfactant in the amount of not less than 0.1 part by mass to 100 parts by mass of the organic acid, preferably not less than 3 parts by mass, more preferably not less than 5 parts by mass, and preferably not more than 20 parts by mass, more preferably not more than 15 parts by mass, to 100 parts by mass of the organic acid, and a water-soluble binder to obtain a mixture;

step 2: granulating, or compress-molding, or compress-molding after granulating, the mixture obtained in step 1 to obtain a granulated or compress-molded product; and

step 3 : mixing the granulated or compress-molded product obtained in step 2 with a carbonate.

[2] the method for producing an effervescent bath agent composition according to [1], wherein the nonionic surfactant has HLB of 7 to 20, preferably 7.5 to 20, more preferably 8 to 20, even more preferably 8 to 18, and still even more preferably  8 to 15;

[3] the method for producing an effervescent bath agent composition according to [1] or [2], wherein an amount of the nonionic surfactant mixed in step 1 with 100 parts by mass of organic acid in step 1 is 0.1 to 20 parts by mass, preferably 1 to 20 parts by mass, more preferably 1 to 15 parts by mass, even more preferably 3 to 15 parts by mass, and still even more preferably 5 to 15 parts by mass;

[4] the method for producing an effervescent bath agent composition according to any one of [1] to [3], further including step 4 of granulating or compress-molding the mixture, obtained in step 3, to form a granulated or compress-molded product;

[5] the method for producing an effervescent bath agent composition according to any one of [1] to [4], wherein step 1 further includes mixing an oil component in an amount of not more than 4 parts by mass to 100 parts by mass of organic acid;

[6] the method for producing an effervescent bath agent composition according to any one of [1] to [5], wherein the water-soluble binder is polyethylene glycol or polypropylene glycol;

[7] the method for producing an effervescent bath agent composition according to any one of [1] to [6], wherein the nonionic surfactant is a fatty acid ester having a polyoxyalkylene group, and preferably a polyoxyethylene sorbitan fatty acid ester;

[8] the method for producing an effervescent bath agent composition according to any one of [1] to [7], wherein the nonionic surfactant is in the form of liquid or paste, preferably in a liquid form at an ambient temperature (25°C);

[9] the method for producing an effervescent bath agent composition according to any one of [1] to [8], wherein a content of the water-soluble binder in the bath agent composition is  2 to 20% by mass, and preferably 5 to 15% by mass;

[10] the method for producing an effervescent bath agent composition according to any one of [1] to [9], wherein a content of the organic acid in the bath agent composition is 30 to 80% by mass, preferably 40 to 70% by mass, and more preferably 40 to 60% by mass;

[11] the method for producing an effervescent bath agent composition according to any one of [1] to [10], wherein a content of the carbonate in the bath agent composition is 20 to 70% by mass, preferably 30 to 60% by mass, and more preferably 30 to 50% by mass;

[12] the method for producing an effervescent bath agent composition according to any one of [1] to [11], wherein a content of the nonionic surfactant in the bath agent composition is 0.5 to 12% by mass, preferably 1 to 10% by mass, and more preferably 2 to 8% by mass;

[13] the method for producing an effervescent bath agent composition according to any one of [1] to [12], wherein the organic acid is at least one acid selected from the group consisting of fumaric acid, succinic acid, malicacid and citric acid;

[14] the method for producing an effervescent bath agent composition according to any one of [1] to [13], wherein the organic acid is at least one of fumaric acid and succinic acid;

[15] the method for producing an effervescent bath agent composition according to any one of [1] to [14], wherein a mass ratio of the organic acid to the carbonate, organic acid/carbonate, is 30/70 to 80/20, preferably 40/60 to 70/30, and more preferably 40/60 to 60/40;

[16] the method for producing an effervescent bath agent composition according to any one of [1] to [15], wherein a content of sodium carbonate in the carbonate is 70 to 100% by mass;

[17] the method for producing an effervescent bath agent composition according to any one of [1] to [16], wherein step 1 further includes mixing an oil component and an anionic surfactant;

[18] An effervescent bath agent composition produced by the method according to any one of [1] to [17];

[19] the effervescent bath agent composition according to [18], which is in the form of tablet, briquette, or powder; and

[20] the effervescent bath agent composition according to [18] or [19], which has pH of 5 to 7, and more preferably 5.5 to 6.5, in an aqueous 0.01% by mass solution at 25°C.

Examples

The following Examples demonstrate the present invention. Examples are intended to illustrate the present invention and not to limit the present invention.

<Methods of measuring physical properties of a bath agent composition>

1. Particle size

Two methods of measuring a particle size were employed.
(1) A particle size of a carbonate was a median diameter determined by screening through standard sieves of JIS K 8801 (mesh opening: 2000 to 125 $\mu$m) with vibration for five minutes, and calculating 50% particle size by mass.

More specifically, this sieve analysis used nine sieves with mesh openings of 125 $\mu$m, 180 $\mu$m, 250 $\mu$m, 355 $\mu$m, 500 $\mu$m, 710 $\mu$m, 1000 $\mu$m, 1400 $\mu$m, and 2000 $\mu$m and a saucer. Nine sieves were stacked on the saucer from the smallest mesh opening to the largest. 100 g of a granular carbonate was added to put the topmost sieve with 2000 $\mu$m mesh opening and covered with a lid. A column of the sieves and the saucer was mounted on a Ro-Tap sieve shaker (Heiko Seisakusyo, tapping: 156 times/min, rolling: 290 rounds/min) and shaken for five minutes. Masses of granules retained on each sieve and the saucer were measured and divided by the total mass to obtain respective percentages by mass (%). by mass

The mass proportions of the particles in the order beginning from the receiving tray to those sieves having smaller sieve openings are cumulated, and a particle size at which a total is 50% mass proportions is defined as an average particle size.

(2) Average particle sizes of an organic acid and a disintegrating agent were a median diameter determined in a dispersed state in a solvent in which they are insoluble with a laser diffraction/scattering particle size distribution analyzer LA-920 (Horiba Ltd.). In order to determine mean particle sizes of fumaric acid and a disintegrating agent used in Examples, acetone was used as the solvent.

2. Oil absorption capacity

An oil absorption capacity of an organic acid was determined by the following method. In an apparatus for measuring an absorption amount (Asahisouken Co., Ltd., model: S410), 45 g of powder sample was charged and agitated with a driving blade at 200 rpm. Here was added a nonionic surfactant (Nikkol GO-440V, HLB = 12.6, manufactured by Nikko Chemicals Co., Ltd.) dropwise at a supply rate of 2 ml/min to determine a point for a maximum torque. An amount of the nonionic surfactant added that resulted 70% torque of the maximum torque was divided by the amount of the powder charged to obtain an oil absorption capacity.

<Method of measuring a distribution ratio of carbon dioxide between an oil phase and an aqueous phase>

Distribution of carbon dioxide was made by the following procedure at an ambient temperature (25°C): inserting a nozzle at the bottom in a 15 mL screw vial containing 2 mL of oil component and 8 mL of ion-exchanged water and injecting a sufficient amount of carbon dioxide continuously over four minutes; stopping the injection and allowing the vial to stand for separating into phases; when an oil phase was formed on the interface, immediately taking a sample of the oil phase from the vial for measuring an amount of carbon dioxide; and gently slipping a pipette in the bottom of the vial and tasking a sample of the aqueous phase for measuring an amount of carbon dioxide.

A concentration of carbon dioxide was measured by ATR-IR spectroscopy. Carbon dioxide is known to have a strong band in its IR spectrum at about 2350 cm$^{-1}$. This absorption band includes one vibration mode. In a gas state, a rotational transition of carbon dioxide is superimposed on the vibration mode, and the absorption accordingly provides two apparent splitting bands in a measurement with a low resolution. In a dissolved state in a liquid, carbon dioxide is restricted in rotation, and the absorption accordingly provides a single band. The difference distinguishes a gaseous carbon dioxide from a dissolved carbon dioxide.

measurement conditions

apparatus: PerkinElmer Spectrum One/Universal ATR unit

resolution: 16 cm$^{-1}$

scanning: 16 times (for about 25 seconds)

atmospheric correction: OFF

measuring order: oil background → oil phase → water background → aqueous phase

<Evaluation of an effervescent bath agent composition> [Fine bubble generation (turbidity)]

On circular filter papers having a 50-mm diameter, "A" was written with an oily black marker (width: 2 mm), within a 30 mm square frame, a 1 L glass beaker (outer diameter of the body: 110 mm, height: 150 mm, IWAKI•PYREX (registered trademark)) containing 1 L of hot water at 40°C or 2 L glass beakers (outer diameter of the body: 135 mm, height: 200 mm, IWAKI•PYREX (registered trademark)) containing 1.5/1.75/2.0/2.25 L of hot water at 40°C, respectively, were placed on the filter papers. 7 g of each bath agent composition of Examples and Comparative Examples was incorporated in the beakers and observed from the side and from above. After one minute from the input of the bath agent composition, fine bubble generation (turbidity) was graded on the following scale. In the scale, an 8 rating is most favorable, and the smaller number is the less favorable.

Rating scale of fine bubble generation (turbidity)

8: In evaluation in a 2 L glass beaker (2.25 L of hot water), a bath agent composition generates fine gas bubbles of carbon dioxide with clouding enough to obscure the letter under the beaker (pass)

7 : In evaluation in a 2 L glass beaker (2.0 L of hot water), a bath agent composition generates fine gas bubbles of carbon dioxide for clouding enough to obscure the letter under the beaker (pass)

6: In evaluation in a 2 L glass beaker (1.75 L of hot water), a bath agent composition generates fine gas bubbles of carbon dioxide for clouding enough to obscure the letter under the beaker (pass)

5 : In evaluation in a 2 L glass beaker (1.5 L of hot water), a bath agent composition generates fine gas bubbles of carbon dioxide for clouding enough to obscure the letter under the beaker (pass)

4: In evaluation in a 2 L glass beaker (1.5 L of hot water), a bath agent composition generates fine gas bubbles of carbon dioxide for clouding, but the letter under the beaker can be faintly seen. Then in evaluation in a 1 L glass beaker (1 L of hot water), the composition generates fine gas bubbles of carbon dioxide for clouding enough to obscure the letter under the beaker (pass)

3: In evaluation in a 1 L glass beaker (1 L of hot water), a bath agent composition generates fine gas bubbles of carbon dioxide for clouding to obscure the letter under the beaker (pass)

2: In evaluation in a 1 L glass beaker (1 L of hot water), a bath agent composition generates fine gas bubbles of carbon dioxide for a weak clouding, and the letter under the beaker can be faintly seen (fail)

1: In evaluation in a 1 L glass beaker (1 L of hot water), a bath agent composition fail to cloud the water with fine gas bubbles, and the letter under the beaker can be clearly seen (fail)

Preparation Example 1

In a 2 L High-speed mixer (Earthtechnica Co. Ltd., model: LFS-2, agitator rotation speed: 200 r.p.m., chopper rotation: 500 r.p.m., Froude number: 1.7, hot-water temperature in a jacket: 60°C), 178 g of fumaric acid-1 and 1 g of tetraoleic acid polyoxyethylene sorbit (with 40 EO groups) were mixed for 1 minute.

To this was added 21 g of polyethylene glycol and mixed for 3 minutes to obtain the mixture.

The resultant mixture was subjected to extrusion granulation with a Dome granulator (Dalton Co., Ltd., model: DG-L1, screen: 0.7 mm opening, rotation speed: 20 rpm).

The extruded granules were laid over a tray, cooled at 25°C, and subjected to size regulation with a Power Mill (Dalton Co., Ltd., model: P-02S, screen: 2.5 mm opening) to obtain granules of Preparation Example 1.

Preparation Example 2

In a 30 L Nauta mixer (Hosokawa Micron Corporation, model: NX-S, axial rotation speed: 100 r.p.m., Froude number: 0. 84, hot-water temperature in a jacket: 60°C), 7500 g of fumaric acid-1 and 300 g of tetraoleic acid polyoxyethylene sorbit (with 40 EO groups) were mixed for 30 minutes.
To this was added 1200 g of polyethylene glycol and mixed for 15 minutes. To this was further added 1000 g of glucose and mixed for 5 minutes to obtain the mixture.
The resultant mixture was subjected to extrusion granulation with a Twin Dome Granulator (Dalton Co., Ltd., model: TDG-110, screen: 0.7 mm opening, rotation speed: 36 rpm) .
The extruded granules were laid over a tray, cooled at 25°C, and subjected to size regulation with a Power Mill (Dalton Co., Ltd., model: P-02S, screen: 2.5 mm opening) to obtain granules of Preparation Example 2.

Preparation Example 3

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-1 and 700 g of tetraoleic acid polyoxyethylene sorbit (with 40 EO groups) were mixed for 30 minutes.
To this was added 1200 g of polyethylene glycol and mixed for 15 minutes. To this was further added 600 g of glucose and mixed for 5 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm).
The extruded granules were laid over a tray, cooled at 25°C, and subjected to size regulation with a Power Mill (Dalton Co., Ltd.., model: P-02S, screen: 2.5 mm opening) to obtain granules of Preparation Example 3.

Preparation Example 4

In a 30 L Nauta mixer (Hosokawa Micron Corporation, model: NX-S, axial rotation speed: 100 r.p.m. , Froude number: 0.84, hot-water temperature in a jacket: 60°C), 7500 g of fumaric acid-2 and 700 g of tetraoleic acid polyoxyethylene sorbit (with 40 EO groups) were mixed for 30 minutes.
To this was added 1200 g of polyethylene glycol and mixed for 15 minutes. To this was further added 600 g of glucose and mixed for 5 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm).
The extruded granules were laid over a tray, cooled at 25°C, and subjected to size regulation with a Power Mill (Dalton Co., Ltd., model: P-02S, screen: 2.5 mm opening) to obtain granules of Preparation Example 4.

Preparation Example 5

In a 30 L Nauta mixer (Hosokawa Micron Corporation, model: NX-S, axial rotation speed: 100 r.p.m. , Froude number: 0.84, hot-water temperature in a jacket: 60°C), 7500 g of fumaric acid-2 and 1000 g of tetraoleic acid polyoxyethylene sorbit (with 40 EO groups) were mixed for 30 minutes.
To this was added 1200 g of polyethylene glycol and mixed  for 15 minutes. To this was further added 300 g of glucose and mixed for 5 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm) .
The extruded granules were laid over a tray, cooled at 25°C, and subjected to size regulation with a Power Mill (Dalton Co., Ltd., model: P-02S, screen: 2.5 mm opening) to obtain granules of Preparation Example 5.

Preparation Example 6

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2 and 700 g of tetraoleic acid polyoxyethylene sorbit (with 40 EO groups) were mixed.
To this was added 1600 g of polyethylene glycol and mixed for 15 minutes. To this was further added 200 g of glucose and mixed for 5 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm).
The extruded granules were laid over a tray, cooled at 25°C, and subjected to size regulation with a Power Mill (Dalton Co., Ltd., model: P-02S, screen: 2.5 mm opening) to obtain granules of Preparation Example 6.

Preparation Example 7

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2 and 700 g of tetraoleic acid polyoxyethylene sorbit (with 40 EO groups) were mixed.
To this was added 1600 g of polyethylene glycol and mixed for 15 minutes. To this was further added 200 g of glucose and mixed for 5 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm).
The extruded granules were laid over a tray, cooled at 25°C, and subjected to size regulation with a Power Mill (Dalton Co., Ltd., model: P-02S, screen: 2.5 mm opening) to obtain granules.
These granules were compress-molded with a briquetter (Sintokogio Ltd., model: BSS501-010, briquetting size: 5 mm) to form briquettes. These briquettes were screened through sieves with 4 mm opening and 5.6 mm opening to obtain briquettes retained on the sieve with 4 mm opening, while passing through the sieve with 5.6 mm opening of Preparation Example 7.

Preparation Example 8

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2 and 700 g of tetraoleic acid polyoxyethylene sorbit (with 40 EO groups) were mixed for 10 minutes.
300 g of isopropyl palmitate was added and mixed for 20 minutes. 1500 g of polyethylene glycol was added and mixed for 15 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm) .
The extruded granules were laid over a tray, cooled at 25°C, and subjected to size regulation with a Power Mill (Dalton Co., Ltd., model: P-02S, screen: 2.5 mm opening) to obtain granules of Preparation Example 8.

Preparation Example 9

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2 and 700 g of polyoxyethylene oleyl ether (with 13 EO groups) heated to 70°C were mixed for 30 minutes.
To this was added 1200 g of polyethylene glycol and mixed for 15 minutes. To this was further added 600 g of glucose and mixed for 5 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm) .
The extruded granules were laid over a tray, cooled at 25°C, and subjected to size regulation with a Power Mill (Dalton Co., Ltd., model: P-02S, screen: 2.5 mm opening) to obtain granules of Preparation Example 9.

Preparation Example 10

In a 30 L Nauta mixer (Hosokawa Micron Corporation, model: NX-S, axial rotation speed: 100 r.p.m., Froude number: 0. 84, hot-water temperature in a jacket: 60°C), 7500 g of fumaric acid -2 and 700 g of polyoxyethylene lauryl ether (with 5 EO groups) were mixed for 30 minutes.
To this was added 1200 g of polyethylene glycol and mixed for 15 minutes. To this was further added 600 g of glucose and mixed for 5 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm).
The extruded granules were laid over a tray, cooled at 25°C, and subjected to size regulation with a Power Mill (Dalton Co., Ltd., model: P-02S, screen: 2.5 mm opening) to obtain granules of Preparation Example 10.

Preparation Example 11

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2 and 700 g of polyoxyethylene sorbitan monooleate (with 6 EO groups) were mixed for 30 minutes.

To this was added 1200 g of polyethylene glycol and mixed for 15 minutes. To this was further added 600 g of glucose and mixed for 5 minutes to obtain the mixture.

The resultant mixture was subjected to extrusion granulation with a Twin Dome granulator (Dalton Co., Ltd., model: TDG-110, screen: 0.7 mm opening, rotation speed: 36 rpm).

The extruded granules were laid over a tray, cooled at 25°C, and subjected to size regulation with a Power Mill (Dalton Co., Ltd., model: P-02S, screen: 2.5 mm opening) to obtain granules of Preparation Example 11.

Preparation Example 12

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2 and 700 g of polyoxyethylene stearyl ether (with 6 EO groups) heated to 70°C were mixed for 30 minutes.

To this was added 1200 g of polyethylene glycol and mixed for 15 minutes. To this was further added 600 g of glucose and mixed for 5 minutes to obtain the mixture.

The resultant mixture was subjected to extrusion granulation with a Twin Dome granulator (Dalton Co., Ltd., model: TDG-110, screen: 0.7 mm opening, rotation speed: 36 rpm).

The extruded granules were laid over a tray, cooled at 25°C, and subjected to size regulation with a Power Mill (Dalton Co., Ltd., model: P-02S, screen: 2.5 mm opening) to obtain granules of Preparation Example 14.

Preparation Example 13

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2 and 700 g of polyoxyethylene sorbitan monooleate (with 20 EO groups) were mixed for 30 minutes.

To this was added 1200 g of polyethylene glycol and mixed for 15 minutes. To this was further added 600 g of glucose and mixed for 5 minutes to obtain the mixture.

The resultant mixture was subjected to extrusion granulation with a Twin Dome granulator (Dalton Co., Ltd., model: TDG-110, screen: 0.7 mm opening, rotation speed: 36 rpm).

The extruded granules were laid over a tray, cooled at 25°C, and subjected to size regulation with a Power Mill (Dalton Co., Ltd., model: P-02S, screen: 2.5 mm opening) to obtain granules of Preparation Example 14.

Preparation Example 14

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2 and 700 g of polyoxyethylene sorbitan monolaurate (with 20 EO groups) were mixed for 30 minutes.

To this was added 1200 g of polyethylene glycol and mixed for 15 minutes. To this was further added 600 g of glucose and mixed for 5 minutes to obtain the mixture.

The resultant mixture was subjected to extrusion granulation with a Twin Dome granulator (Dalton Co., Ltd., model: TDG-110, screen: 0.7 mm opening, rotation speed: 36 rpm).

The extruded granules were laid over a tray, cooled at 25°C, and subjected to size regulation with a Power Mill (Dalton Co., Ltd., model: P-02S, screen: 2.5 mm opening) to obtain granules of Preparation Example 14.

Preparation Example 15

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2 and 700 g of polyoxyethylene lauryl ether (with 41 EO groups) heated to 70°C were mixed for 30 minutes.

To this was added 1200 g of polyethylene glycol and mixed for 15 minutes. To this was further added 600 g of glucose and mixed for 5 minutes to obtain the mixture.

The resultant mixture was subjected to extrusion granulation with a Twin Dome granulator (Dalton Co., Ltd., model: TDG-110, screen: 0.7 mm opening, rotation speed: 36 rpm).

The extruded granules were laid over a tray, cooled at 25°C, and subjected to size regulation with a Power Mill (Dalton Co., Ltd., model: P-02S, screen: 2.5 mm opening) to obtain granules of Preparation Example 15.

Preparation Example 16

In a 30 L Nauta mixer (Hosokawa Micron Corporation, model: NX-S, axial rotation speed: 100 r.p.m., Froude number: 0.84, hot-water temperature in a jacket : 60°C), 7500 g of fumaric acid-2 and 700 g of tetraoleic acid polyoxyethylene sorbit (with 40 EO groups) were mixed for 10 minutes.
To this was added 200 g of isopropyl palmitate and mixed for 20 minutes.
To this was further added 1500 g of polyethylene glycol and mixed for 15 minutes. To this was further added 100 g of glucose and mixed for 5 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm).
The extruded granules were laid over a tray, cooled at 25°C, and subjected to size regulation with a Power Mill (Dalton Co., Ltd., model: P-02S, screen: 2.5 mm opening) to obtain granules of Preparation Example 16.

Preparation Example 17

In a 30 L Nauta mixer (Hosokawa Micron Corporation, model: NX-S, axial rotation speed: 100 r.p.m., Froude number: 0.84, hot-water temperature in a jacket: 60°C), 7500 g of fumaric acid-2, 700 g of tetraoleic acid polyoxyethylene sorbit (with 40 EO groups), and 50 g of polyoxyethylene tridecyl ether acetic acid sodium salt (with 3 EO groups) were mixed for 10 minutes.
To this was added 200 g of isopropyl palmitate and mixed for 20 minutes.
To this was further added 1500 g of polyethylene glycol and mixed for 15 minutes. To this was further added 50 g of glucose and mixed for 5 minutes to obtain the mixture.
The resultant mixture was subjected to extrusion granulation with a Twin Dome granulator (Dalton Co., Ltd., model: TDG-110, screen: 0.7 mm opening, rotation speed: 36 rpm).
The extruded granules were laid over a tray, cooled at 25°C, and subjected to size regulation with a Power Mill (Dalton Co., Ltd., model: P-02S, screen: 2.5 mm opening) to obtain granules of Preparation Example 17.

Comparative Preparation Example 1

In a 2 L High-speed mixer (Earthtechnica Co.Ltd. , model: LFS-2, agitator rotation speed: 200 r.p.m., chopper rotation: 500 r.p.m., Froude number: 1.7, hot-water temperature in a jacket: 60°C), 178 g of fumaric acid-1 and 22 g of polyethylene glycol were mixed for 3 minutes to obtain the mixture.
The resultant mixture was subjected to extrusion granulation with a Dome granulator (Dalton Co., Ltd., model: DG-L1, screen: 0.7 mm opening, rotation speed: 20 rpm).
The extruded granules were laid over a tray, cooled at 25°C, and subjected to size regulation with a Power Mill (Dalton Co., Ltd., model: P-02S, screen: 2.5 mm opening) to obtain granules of Comparative Preparation Example 1.

EP 2 664 319 A1

[Table 1]

| | | | Preparation example | | | | | | | | Comparative preparation example |
|---|---|---|---|---|---|---|---|---|---|---|---|
| | | | 1 | 2 | 3 | 4 | 5 | 6 | 7 | 8 | 1 |
| Granulated or compress-molded product(parts by mass) | Organic acid | Fumaric acid −1 | 89 | 75 | 75 | − | − | − | − | − | 89 |
| | | Fumaric acid −2 | − | − | − | 75 | 75 | 75 | 75 | 75 | − |
| | Nonionic surfactant | Tetraoleic acid polyoxyethylene sorbit (with 40 EO groups) HLB=10.1 | 0.5 | 3 | 7 | 7 | 10 | 7 | 7 | 7 | − |
| | Water-soluble binder | Polyethylene glycol | 10.5 | 12 | 12 | 12 | 12 | 16 | 16 | 15 | 11 |
| | Oily component | Isopropyl palmitate | − | − | − | − | − | − | − | 3 | − |
| | Disintegrating agent | Glucose | − | 10 | 6 | 6 | 3 | 2 | 2 | − | − |
| | the total | | 100 | 100 | 100 | 100 | 100 | 100 | 100 | 100 | 100 |
| Mixer | | | High-speed mixer | Nauta mixer | Nauta mixer | Nauta mixer | Nauta mixer | Nauta mixer | Nauta mixer | Nauta mixer | High-speed mixer |
| Operation | | | Extrusion granulation | Extrusion granulation | Extrusion granulation | Extrusion granulation | Extrusion granulation | Extrusion granulation | Extrusion granulation → briquetting | Extrusion granulation | Extrusion granulation |
| Form | | | Granule | Granule | Granule | Granule | Granule | Granule | Briquette | Granule | Granule |

16

In Preparation Examples and Comparative Preparation Examples, the following materials were used.

[Table 2]

| | | | Preparation example | | | | | | | | |
|---|---|---|---|---|---|---|---|---|---|---|---|
| | | | 9 | 10 | 11 | 12 | 13 | 14 | 15 | 16 | 17 |
| Organic acid | | Fumaric acid -1 | | | | | | | | – | – |
| | | Fumaric acid -2 | 75 | 75 | 75 | 75 | 75 | 75 | 75 | 75 | 75 |
| Nonionic surfactant | | Tetraoleic acid polyoxyethylene sorbit (with 40 EO groups) HLB=10.1 | | | | | | | | 7 | 7 |
| | | Polyoxyethylene oleyl ether (with 13 EO groups) HLB=10.9 | 7 | | | | | | | | |
| | | Polyoxyethylene lauryl ether (with 5 EOgroups) HLB=8.0 | | 7 | | | | | | | |
| | | Polyoxyethylene sorbitan monoleate (with 6 EO groups) HLB=9.6 | | | 7 | | | | | | |
| | | Polyoxyethylene stearyl ether (with 6 EO groups) HLB=7.0 | | | | 7 | | | | | |
| | | Polyoxyethylene sorbitan monooleate (with 20 EO groups) HLB=14.0 | | | | | 7 | | | | |
| | | Polyoxyethylene sorbitan monolauleate (with 20 EO groups) HLB=15.9 | | | | | | 7 | | | |
| | | Polyoxyethylene lauryl ether (with 41 EO groups) HLB=18.6 | | | | | | | 7 | | |
| Anionic surfactant | | Polyoxyethylene tridecyl ether acetic acid sodium salt (with 3 EO groups) | | | | | | | | | 0.5 |
| Water-soluble binder | | Polyethylene glycol | 12 | 12 | 12 | 12 | 12 | 12 | 12 | 15 | 15 |
| Oil component | | Isopropyl palmitate | – | – | – | – | – | – | – | 2 | 2 |
| Disintefrating agent | | Glucose | 6 | 6 | 6 | 6 | 6 | 6 | 6 | 1 | 0.5 |
| the total | | | 100 | 100 | 100 | 100 | 100 | 100 | 100 | 100 | 100 |
| Mixer | | | Nauta mixer | Nauta mixer | Nauta mixer | Nauta mixer | Nauta mixer | Nauta mixer | Nauta mixer | Nauta mixer | Nauta mixer |
| Operation | | | Extrusion granulation | Extrusion granulation | Extrusion granulation | Extrusion granulation | Extrusion granulation | Extrusion granulation | Extrusion granulation | Extrusion granulation | Extrusion granulation |
| Form | | | Granule | Granule | Granule | Granule | Granule | Granule | Granule | Granule | Granule |

(Granule or compressed product)(parts by mass)

(organic acid)

- fumaric acid-1: commercial product available from Nippon Shokubai Co., Ltd. , oil absorption capacity: 0.112 mL/g, a mean particle size: 140 $\mu$m
- fumaric acid-2: prepared by pulverizing a commercial product available from Nippon Shokubai Co., Ltd., oil absorption capacity: 0.286 mL/g, a mean particle size: 35 $\mu$m (nonionic surfactant) The added mole number of EO groups is an average added mole number.
- tetraoleic acid polyoxyethylene sorbit (with 40 EO groups) : Nikkol GO440V available from Nikko Chemicals Co., Ltd., liquid at ambient temperature, HLB = 10.1 according to the Kawakami's method, in which the total molecular mass of hydrophilic portions is [sorbitol moiety ($C_6H_{10}O_6$)] + [polyoxyethylene moiety ($C_2H_4O$)ra40] = 1938, and the total molecular mass of lipophilic portions is [tetraoleate moiety (($C_{18}H_{33}O$) x 4)] = 1060, and accordingly HLB = 7 + 11.7 log (1938/1060).
- polyoxyethylene oleyl ether (with 13 EO groups):

  Emulgen 420 available from Kao Corporation, paste at ambient temperature HLB = 10.9 according to the Kawakami's method, in which the total molecular mass of hydrophilic portions is [polyoxyethylene moiety (($C_2H_4O$) ra13 + 1)] = 573, and the total molecular mass of lipophilic portions is [oleyl alcohol moiety ($C_{18}H_{33}O$)] = 265, and accordingly HLB = 7 + 11.7 log (573/265).

- polyoxyethylene lauryl ether (with 5 EO groups) :

  Emulgen 106 available from Kao Corporation, liquid at ambient temperature, HLB = 8.0.

- polyoxyethylene sorbitan monooleate (with 6 EO groups) Rheodol TW-O 106V available from Kao Corporation, liquid at ambient temperature, HLB = 9.6.
- polyoxyethylene stearyl ether (with 6 EO groups) :

  Emulgen 306V available from Kao Corporation, wax at ambient temperature, HLB = 7.0.

- polyoxyethylene sorbitan monooleate (with 20 EO groups) : Rheodol TW-O 120V available from Kao Corporation, liquid at ambient temperature HLB = 14.0.
- polyoxyethylene sorbitan monolaurate (with 20 EO groups) : Rheodol TW-L 120 available from Kao Corporation, liquid at ambient temperature, HLB = 15.9.
- polyoxyethylene lauryl ether (with 41 EO groups) :

  Emulgen 130K available from Kao Corporation, flakes at ambient temperature, HLB = 18.6.

(water-soluble binder)

- polyethylene glycol: K-PEG 6000LA available from Kao Corporation, number average-molecular weight: 8500

(oil component)

- isopropyl palmitate: Exceparl IPP available from Kao Corporation

(anionic surfactant)

- polyoxyethylene tridecyl ether acetic acid sodium salt (with 3 EO groups): Nikkol ECTD-3NEX available from Nikko Chemicals Co., Ltd.

(disintegrating agent)

- glucose: anhydrous glucose crystalline #900 available from Nihon Shokuhin Kako Co., Ltd., a mean particle size: 25 $\mu$m

(carbonate)

- sodium carbonate: Dense soda ash available from Central Glass Co., Ltd., a mean particle size: 290 $\mu$m

Example 1

In a 2.5 L Nauta mixer (Hosokawa Micron Corporation, model: LABOMIXER LV-1, rotation speed: set to a maximum scale value, without a jacket), 120 g of granules of Preparation Example 1 and 80 g of sodium carbonate were mixed for 5 minutes to obtain an effervescent bath agent composition in a powder form.
The composition of Example 1 had 4 rating on the evaluation of fine bubble generation.

Example 2

In a 2.5 L Nauta mixer (Hosokawa Micron Corporation, model: LABOMIXER LV-1, rotation speed: set to a maximum scale value, without a jacket), 120 g of granules of Preparation Example 2 and 80 g of sodium carbonate were mixed for 5 minutes to obtain an effervescent bath agent composition in a powder form.
The composition of Example 2 had 5 rating on the evaluation of fine bubble generation.

Example 3

In a 2.5 L Nauta mixer (Hosokawa Micron Corporation, model: LABOMIXER LV-1, rotation speed: set to a maximum scale value, without a jacket), 120 g of granules of Preparation Example 3 and 80 g of sodium carbonate were mixed for 5 minutes to obtain an effervescent bath agent composition in a powder form.
The composition of Example 3 had 6 rating on the evaluation of fine bubble generation. Comparison among Examples 1 to 3 showed an increasing fine bubble generation with an increasing amount of nonionic surfactant added.

Example 4

In a 2.5 L Nauta mixer (Hosokawa Micron Corporation, model: LABOMIXER LV-1, rotation speed: set to a maximum scale  value, without a jacket), 120 g of granules of Preparation Example 4 and 80 g of sodium carbonate were mixed for 5 minutes to obtain an effervescent bath agent composition in a powder form.
The composition of Example 4 had 7 rating on the evaluation of fine bubble generation. Comparison between Examples 3 and 4 showed that the organic acid having the smaller average particle size resulted in the greater fine bubble generation (higher turbidity).

Example 5

In a 2.5 L Nauta mixer (Hosokawa Micron Corporation, model: LABOMIXER LV-1, rotation speed: set to a maximum scale value, without a jacket), 120 g of granules of Preparation Example 5 and 80 g of sodium carbonate were mixed for 5 minutes to obtain an effervescent bath agent composition in a powder form.
The composition of Example 5 had 8 rating on the evaluation of fine bubble generation. Comparison between Examples 4 and 5 showed that the larger amount of nonionic surfactant resulted in the greater fine bubble generation.

Example 6

In a 2.5 L Nauta mixer (Hosokawa Micron Corporation, model: LABOMIXER LV-1, rotation speed: set to a maximum scale value, without a jacket), 120 g of granules of Preparation Example 6 and 80 g of sodium carbonate were mixed for 5 minutes to obtain an effervescent bath agent composition in a powder form.
The composition of Example 6 had 7 rating on the evaluation of fine bubble generation.

Example 7

In a 2.5 L Nauta mixer (Hosokawa Micron Corporation, model: LABOMIXER LV-1, rotation speed: set to a maximum scale value, without a jacket), 120 g of briquettes of Preparation Example 7 and 80 g of sodium carbonate were mixed for 5 minutes to obtain an effervescent bath agent composition in a granule form (briquette/powder mixture).
The composition of Example 7 had 5 rating on the evaluation of fine bubble generation.

Example 8

In a 2.5 L Nauta mixer (Hosokawa Micron Corporation, model: LABOMIXER LV-1, rotation speed: set to a maximum scale value, without a jacket), 300 g of granules of Preparation Example 6 and 200 g of sodium carbonate were mixed for 5 minutes to obtain a mixture.

The resultant mixture was compress-molded with a briquetter (Sintokogio Ltd., model: BSS501-010, briquetting size: 5 mm) to form briquettes.

The resultant briquettes were screened through sieves with 4 mm opening and 5.6 mm opening to obtain briquettes retained on the sieve with 4 mm opening, while passing through the sieve with 5.6 mm opening. These briquettes were used as an effervescent bath agent composition.

The composition of Example 8 had 5 rating on the evaluation of fine bubble generation.

Example 9

In a 2.5 L Nauta mixer (Hosokawa Micron Corporation, model: LABOMIXER LV-1, rotation speed: set to a maximum scale value, without a jacket), 120 g of granules of Preparation Example 6 and 80 g of sodium carbonate were mixed for 5 minutes to obtain an effervescent bath agent composition. 7 g of the  resultant composition was filled in a tableting cell with a diameter of 25 mm and compress-molded under 20 MPa with a tableting machine (Riken) to form a tablet of the effervescent bath agent composition having dimensions of 25 mm diameter and 7 mm thickness.

The composition of Example 9 had 4 rating on the evaluation of fine bubble generation. It is noted in comparison of Examples 6 to 9 that generation of fine bubbles was better, according to the form of a bath agent composition, in order of from powder, briquette, and then tablet.

Example 10

In a 2.5 L Nauta mixer (Hosokawa Micron Corporation, model: LABOMIXER LV-1, rotation speed: set to a maximum scale value, without a jacket), 120 g of granules of Preparation Example 8 and 80 g of sodium carbonate were mixed for 5 minutes to obtain an effervescent bath agent composition in a powder form.

The composition of Example 10 had 5 rating on the evaluation of fine bubble generation. Comparison between Examples 6 and 10 showed that addition of the oil component resulted in decreased fine bubble generation (decreased turbidity).

Example 11

In a 2.5 L Nauta mixer (Hosokawa Micron Corporation, model: LABOMIXER LV-1, rotation speed: set to a maximum scale value, without a jacket), 300 g of granules of Preparation Example 8 and 200 g of sodium carbonate were mixed for 5 minutes to obtain a mixture.

The resultant mixture was compress-molded with a briquetter (Sintokogio Ltd., model: BSS501-010, briquetting size: 5 mm) to form briquettes.

The resultant briquettes were screened through sieves with 4 mm opening and 5.6 mm opening to obtain briquettes retained on the sieve with 4 mm opening, while passing through the sieve with 5.6 mm opening. These briquettes were used as an effervescent bath agent composition.

The composition of Example 11 had 4 rating on the evaluation of fine bubble generation. Comparison between Examples 8 and 11 showed that addition of the oil component resulted in decreased fine bubble generation (decreased turbidity).

Example 12

In a 2.5 L Nauta mixer (Hosokawa Micron Corporation, model: LABOMIXER LV-1, rotation speed: set to a maximum scale value, without a jacket), 120 g of granules of Preparation Example 8 and 80 g of sodium carbonate were mixed for 5 minutes to obtain an effervescent bath agent composition. 7 g of the resultant composition was filled in a tableting cell with a diameter of 25 mm and compress-molded under 20 MPa with a tableting machine (Riken) to form a tablet of the effervescent bath agent composition having dimensions of 25 mm diameter and 7 mm thickness.

The composition of Example 12 had 3 rating on the evaluation of fine bubble generation. Comparison between Examples 9 and 12 showed that addition of the oil component resulted in decreased fine bubble generation (decreased turbidity).

Example 13

In a 2.5 L Nauta mixer (Hosokawa Micron Corporation, model: LABOMIXER LV-1, rotation speed: set to a maximum scale value, without a jacket), 120 g of granules of Preparation  Example 9 and 80 g of sodium carbonate were mixed for 5 minutes to obtain an effervescent bath agent composition in a powder form.

The composition of Example 13 had 7 rating on the evaluation of fine bubble generation.

Example 14

In a 2.5 L Nauta mixer (Hosokawa Micron Corporation, model: LABOMIXER LV-1, rotation speed: set to a maximum scale value, without a jacket), 120 g of granules of Preparation Example 10 and 80 g of sodium carbonate were mixed for 5 minutes to obtain an effervescent bath agent composition in a powder form.
The composition of Example 14 had 8 rating on the evaluation of fine bubble generation.

Example 15

In a 2.5 L Nauta mixer (Hosokawa Micron Corporation, model: LABOMIXER LV-1, rotation speed: set to a maximum scale value, without a jacket), 120 g of granules of Preparation Example 11 and 80 g of sodium carbonate were mixed for 5 minutes to obtain an effervescent bath agent composition in a powder form.
The composition of Example 15 had 8 rating on the evaluation of fine bubble generation.

Example 16

In a 2.5 L Nauta mixer (Hosokawa Micron Corporation, model: LABOMIXER LV-1, rotation speed: set to a maximum scale value, without a jacket), 120 g of granules of Preparation Example 12 and 80 g of sodium carbonate were mixed for 5 minutes to obtain an effervescent bath agent composition in a powder form.
The composition of Example 16 had 3 rating on the evaluation of fine bubble generation.

Example 17

In a 2.5 L Nauta mixer (Hosokawa Micron Corporation, model: LABOMIXER LV-1, rotation speed: set to a maximum scale value, without a jacket), 120 g of granules of Preparation Example 13 and 80 g of sodium carbonate were mixed for 5 minutes to obtain an effervescent bath agent composition in a powder form.
The composition of Example 17 had 7 rating on the evaluation of fine bubble generation.

Example 18

In a 2.5 L Nauta mixer (Hosokawa Micron Corporation, model: LABOMIXER LV-1, rotation speed: set to a maximum scale value, without a jacket), 120 g of granules of Preparation Example 14 and 80 g of sodium carbonate were mixed for 5 minutes to obtain an effervescent bath agent composition in a powder form.
The composition of Example 18 had 6 rating on the evaluation of fine bubble generation.

Example 19

In a 2.5 L Nauta mixer (Hosokawa Micron Corporation, model: LABOMIXER LV-1, rotation speed: set to a maximum scale value, without a jacket), 120 g of granules of Preparation Example 15 and 80 g of sodium carbonate were mixed for 5 minutes to obtain an effervescent bath agent composition in a powder form.
The composition of Example 19 had 6 rating on the evaluation of fine bubble generation.

Example 20

In a 2.5 L Nauta mixer (Hosokawa Micron Corporation, model: LABOMIXER LV-1, rotation speed: set to a maximum scale value, without a jacket), 120 g of granules of Preparation Example 16 and 80 g of sodium carbonate were mixed for 5 minutes to obtain an effervescent bath agent composition in a powder form.
The composition of Example 20 had 6 rating on the evaluation of fine bubble generation. Comparison between Examples 10 and 20 showed that the smaller amount of the oil component resulted in the greater fine bubble generation (higher turbidity).

Example 21

In a 2.5 L Nauta mixer (Hosokawa Micron Corporation, model: LABOMIXER LV-1, rotation speed: set to a maximum scale value, without a jacket), 300 g of granules of Preparation Example 16 and 200 g of sodium carbonate were mixed for 5 minutes to obtain a mixture.
The resultant mixture was compress-molded with a briquetter (Sintokogio Ltd., model: BSS501-010, briquetting size: 5 mm) to form briquettes.

The resultant briquettes were screened through sieves with 4 mm opening and 5.6 mm opening to obtain briquettes retained on the sieve with 4 mm opening while passing through the sieve with 5.6 mm opening. These briquettes were used as an effervescent bath agent composition.

The composition of Example 21 had 5 rating on the evaluation of fine bubble generation.

Example 22

In a 2.5 L Nauta mixer (Hosokawa Micron Corporation, model: LABOMIXER LV-1, rotation speed: set to a maximum scale value, without a jacket), 120 g of granules of Preparation Example 16 and 80 g of sodium carbonate were mixed for 5 minutes to obtain an effervescent bath agent composition. 7 g of the resultant composition was put in a tableting cell with a diameter of 25 mm and compress-molded under 20 MPa with a tableting machine (Riken) to form a tablet of the effervescent bath agent composition having dimensions of 25 mm diameter and 7 mm thickness.

The composition of Example 22 had 4 rating on the evaluation of fine bubble generation.

Example 23

In a 2.5 L Nauta mixer (Hosokawa Micron Corporation, model: LABOMIXER LV-1, rotation speed: set to a maximum scale value, without a jacket), 120 g of granules of Preparation Example 17 and 80 g of sodium carbonate were mixed for 5 minutes to obtain an effervescent bath agent composition in a powder form.

The composition of Example 23 had 7 rating on the evaluation of fine bubble generation. Comparison between Examples 20 and 23 showed that addition of the anion surfactant resulted in increased fine bubble generation (increased turbidity).

Example 24

In a 2.5 L Nauta mixer (Hosokawa Micron Corporation, model: LABOMIXER LV-1, rotation speed: set to a maximum scale value, without a jacket), 300 g of granules of Preparation Example 17 and 200 g of sodium carbonate were mixed for 5 minutes to obtain a mixture.

The resultant mixture was compress-molded with a briquetter (Sintokogio Ltd., model: BSS501-010, briquetting size: 5 mm) to form briquettes.

The resultant briquettes were screened through sieves with 4 mm opening and 5.6 mm opening to obtain briquettes retained on the sieve with 4 mm opening, while passing through the sieve with 5.6 mm opening.

The composition of Example 24 had 6 rating on the evaluation of fine bubble generation.

Example 25

In a 2.5 L Nauta mixer (Hosokawa Micron Corporation, model: LABOMIXER LV-1, rotation speed: set to a maximum scale value, without a jacket), 120 g of granules of Preparation Example 17 and 80 g of sodium carbonate were mixed for 5 minutes to obtain an effervescent bath agent composition. 7 g of the resultant composition was put in a tableting cell with a diameter of 25 mm and compress-molded under 20 MPa with a tableting machine (Riken) to form a tablet of the effervescent bath agent composition having dimensions of 25 mm diameter and 7 mm thickness.

The composition of Example 25 had 5 rating on the evaluation of fine bubble generation.

Comparative Example 1

In a 2.5 L Nauta mixer (Hosokawa Micron Corporation, model: LABOMIXER LV-1, rotation speed: set to a maximum scale value, without a jacket), 120 g of granules of Comparative Preparation Example 1 and 80 g of sodium carbonate were mixed for 5 minutes to obtain an effervescent bath agent composition in a powder form.

The composition of Comparative Example 1 had 1 rating on the evaluation of fine bubble generation. It was proved that an organic acid without carrying a nonionic surfactant could not provide fine bubble generation.

Comparative Example 2

In a 2.5 L Nauta mixer (Hosokawa Micron Corporation, model: LABOMIXER LV-1, rotation speed: set to a maximum scale value, without a jacket), 79.4 g of sodium carbonate and 0.6 g of tetraoleic acid polyoxyethylene sorbit (with 40 EO groups) were mixed for 5 minutes. To the mixture was added 120 g of granules of Comparative Preparation Example 1 and mixed for 3 minutes to obtain an effervescent bath agent composition in a powder form.

The composition of Comparative Example 2 had 2 rating on the evaluation of fine bubble generation. It was proved that a carbonate carrying a nonionic surfactant resulted in a decreased amount of fine bubbles.

EP 2 664 319 A1

[Table 3]

| Effervescent bath agent composition | | Example | | | | | | | | | | | | Comparative example | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 1 | 2 |
| Granule or compressed product (the number in parentheses refers to a percentage by mass) | | Production example 1 (60.0) | Production example 2 (60.0) | Production example 3 (60.0) | Production example 4 (60.0) | Production example 5 (60.0) | Production example 6 (60.0) | Production example 6 (60.0) | Production example 6 (60.0) | Production example 6 (60.0) | Production example 8 (60.0) | Production example 8 (60.0) | Production example 8 (60.0) | Comparative production example 1 (60.0) | Comparative production example 1 (60.0) |
| Composition (% by mass) | HLB of nonionic surfactant | 10.1 | 10.1 | 10.1 | 10.1 | 10.1 | 10.1 | 10.1 | 10.1 | 10.1 | 10.1 | 10.1 | 10.1 | 10.1 | |
| | Carbonate *1 | 40.0 | 40.0 | 40.0 | 40.0 | 40.0 | 40.0 | 40.0 | 40.0 | 40.0 | 40.0 | 40.0 | 40.0 | 40.0 | 39.7 |
| | Nonionic surfactant *2 | | | | | | | | | | | | | | 0.3 |
| | the total | 100 | 100 | 100 | 100 | 100 | 100 | 100 | 100 | 100 | 100 | 100 | 100 | 100 | 100 |
| Operation | | Mixing | Mixing | Mixing | Mixing | Mixing | Mixing | Mixing | mixing → briquetti ng | mixing→ briquettin g | Mixing briquetting | mixing→ briquetting ng | mixing→ tabletti ng | Mixing | Mixing |
| Form | | powder | powder | powder | powder | powder | powder | Briquette/Powder | Briquette | Briquette | powder | Briquette | Tablet | powder | powder |
| Fine bubble generation | | 4 | 5 | 6 | 7 | 8 | 7 | 5 | 5 | 4 | 5 | 4 | 3 | 1 | 2 |

*1 Sodium carbonate(Dense soda ash available from Central Glass Co., Ltd., average particle size. 290 μm)

*2 polyoxyethylene sorbit tetraoleate (with 40 EO groups) (Nikko GO440V available from Nikko Chemicals Co.,Ltd., HLB= 10.1)

[Table 4]

| | | Example |||||||||||||
|---|---|---|---|---|---|---|---|---|---|---|---|---|---|---|
| | | 13 | 14 | 15 | 16 | 17 | 18 | 19 | 20 | 21 | 22 | 23 | 24 | 25 |
| Effervescent bath agent composition | Composition (% by mass) | | | | | | | | | | | | | |
| | Granule or compressed product (the number in parenthesis refers to a percentage by mass) | Production example 9 (60.0) | Production example 10 (60.0) | Production example 11 (60.0) | Production example 12 (60.0) | Production example 13 (60.0) | Production example 14 (60.0) | Production example 15 (60.0) | Production example 16 (60.0) | Production example 16 (60.0) | Production example 16 (60.0) | Production example 17 (60.0) | Production example 17 (60.0) | Production example 17 (60.0) |
| | HLB of nonionic surfactant | 10.9 | 8.0 | 9.6 | 7.0 | 14.0 | 15.9 | 18.6 | 10.1 | 10.1 | 10.1 | 10.1 | 10.1 | 10.1 |
| | Carbonate *1 | 40.0 | 40.0 | 40.0 | 40.0 | 40.0 | 40.0 | 40.0 | 40.0 | 40.0 | 40.0 | 40.0 | 40.0 | 40.0 |
| | the total | 100 | 100 | 100 | 100 | 100 | 100 | 100 | 100 | 100 | 100 | 100 | 100 | 100 |
| Operation | | Mixing | Mixing | Mixing | Mixing | Mixing | Mixing | Mixing | Mixing | mixing→briquetting | mixing→tabletting | Mixing | mixing→briquetting | mixing→tabletting |
| Form | | Powder | Powder | Powder | Powder | Powder | Powder | Powder | Powder | Powder briquette | Tablet | Powder | briquette | Tablet |
| Fine bubble generation | | 7 | 8 | 8 | 3 | 7 | 8 | 6 | 6 | 5 | 4 | 7 | 6 | 5 |

*1 Sodium carbonate (Dense soda ash available from Central Glass Co., Ltd., average particle size: 290 $\mu$m)

In Examples and Comparative Examples, the same sodium carbonate was used as in Preparation Examples.

**Claims**

1. A method for producing an effervescent bath agent composition, comprising the following steps:

   step 1: mixing an organic acid, a nonionic surfactant in the amount of not less than 0.1 part by mass, to 100 parts by mass of the organic acid, and a water-soluble binder to obtain a mixture;
   step 2: granulating, or compress-molding, or granulating and then compress-molding, the mixture obtained in step 1 to obtain a granulated or compress-molded product; and
   step 3: mixing the granulated or compress-molded product obtained in step 2 with a carbonate.

2. The method for producing an effervescent bath agent composition according to claim 1, wherein the nonionic surfactant has an HLB of 7 to 20.

3. The method for producing an effervescent bath agent composition according to claim 1 or 2, wherein the amount of the nonionic surfactant, mixed in step 1, is 1 to 20 parts by mass to 100 parts by mass of the organic acid.

4. The method for producing an effervescent bath agent composition according to any one of claims 1 to 3, further comprising step 4 of granulating or compress-molding the mixture, obtained in step 3, to obtain a granulated or compress-molded product.

5. The method for producing an effervescent bath agent composition according to any one of claims 1 to 4, wherein, in step 1, an oil component is further mixed in the amount of not more than 4 parts by mass to 100 parts by mass of organic acid.

6. The method for producing an effervescent bath agent composition according to any one of claims 1 to 5, wherein the water-soluble binder is polyethylene glycol or polypropylene glycol.

7. The method for producing an effervescent bath agent composition according to any one of claims 1 to 6, wherein the nonionic surfactant is a fatty acid ester having a polyoxyalkylene group.

8. The method for producing an effervescent bath agent composition according to any one of claims 1 to 7, wherein the nonionic surfactant is in the form of liquid or paste at an ambient temperature (25°C).

9. The method for producing an effervescent bath agent composition according to any one of claims 1 to 8, wherein the content of the water-soluble binder in the bath agent composition is 2 to 20% by mass.

10. The method for producing an effervescent bath agent composition according to any one of claims 1 to 9, wherein the content of the organic acid in the bath agent composition is 30 to 80% by mass.

11. The method for producing an effervescent bath agent composition according to any one of claims 1 to 10, wherein the content of the carbonate in the bath agent composition is 20 to 70% by mass.

12. The method for producing an effervescent bath agent composition according to any one of claims 1 to 11, wherein the content of the nonionic surfactant in the bath agent composition is 0.5 to 12% by mass.

13. The method for producing an effervescent bath agent composition according to any one of claims 1 to 12, wherein the organic acid is at least one acid selected from the group consisting of fumaric acid, succinic acid, malic acid and citric acid.

14. The method for producing an effervescent bath agent composition according to any one of claims 1 to 13, wherein the organic acid is fumaric acid and/or succinic acid.

15. The method for producing an effervescent bath agent composition according to any one of claims 1 to 14, wherein a mass ratio of the organic acid to the carbonate, organic acid/carbonate, is 30/70 to 80/20.

16. The method for producing an effervescent bath agent composition according to any one of claims 1 to 15, wherein the content of sodium carbonate in the carbonate is 70 to 100% by mass.

**17.** The method for producing an effervescent bath agent composition according to any one of claims 1 to 16, wherein, in step 1, an oil component and an anionic surfactant are further mixed.

**18.** An effervescent bath agent composition, obtained by the method according to any one of claims 1 to 17.

**19.** The effervescent bath agent composition according to claim 18, which is in the form of tablets, briquettes or powder.

**20.** The effervescent bath agent composition according to claim 18 or 19, which has an pH of 5 to 7 in the form of an aqueous 0.01% by mass solution thereof at 25°C.

<table>
<tr><td colspan="2" style="text-align:center"><b>INTERNATIONAL SEARCH REPORT</b></td><td>International application No.<br><br>PCT/JP2012/050327</td></tr>
</table>

**A. CLASSIFICATION OF SUBJECT MATTER**
*A61K8/36*(2006.01)i, *A61K8/19*(2006.01)i, *A61K8/86*(2006.01)i, *A61Q19/10* (2006.01)i

According to International Patent Classification (IPC) or to both national classification and IPC

**B. FIELDS SEARCHED**

Minimum documentation searched (classification system followed by classification symbols)
A61K8/36, A61K8/19, A61K8/86, A61Q19/10

Documentation searched other than minimum documentation to the extent that such documents are included in the fields searched
Jitsuyo Shinan Koho          1922–1996   Jitsuyo Shinan Toroku Koho    1996–2012
Kokai Jitsuyo Shinan Koho    1971–2012   Toroku Jitsuyo Shinan Koho    1994–2012

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| A | JP 1-238520 A  (Kao Corp.),<br>22 September 1989 (22.09.1989),<br>entire text<br>& US 5002758 A          & EP 333223 A2 | 1-20 |
| A | JP 61-176519 A  (Kao Corp.),<br>08 August 1986 (08.08.1986),<br>entire text<br>(Family: none) | 1-20 |
| A | JP 4-128218 A  (Yugen Kaisha Nonokawa Shoji),<br>28 April 1992 (28.04.1992),<br>entire text<br>(Family: none) | 1-20 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>02 March, 2012 (02.03.12) | Date of mailing of the international search report<br>13 March, 2012 (13.03.12) |
| Name and mailing address of the ISA/<br>Japanese Patent Office | Authorized officer |
| Facsimile No. | Telephone No. |

Form PCT/ISA/210 (second sheet) (July 2009)

**EP 2 664 319 A1**

| INTERNATIONAL SEARCH REPORT | | International application No. |
|---|---|---|
| | | PCT/JP2012/050327 |

C (Continuation).    DOCUMENTS CONSIDERED TO BE RELEVANT

| Category* | Citation of document, with indication, where appropriate, of the relevant passages | Relevant to claim No. |
|---|---|---|
| P,X<br>P,A | JP 2011-21004 A  (Kao Corp.),<br>03 February 2011 (03.02.2011),<br>claims; paragraphs [0008], [0013] to [0037],<br>[0051] to [0054], [0056] to [0071]; examples<br>& JP 2011-21005 A | 1-16,18-20<br>17 |
| P,X | JP 2012-1469 A  (Kao Corp.),<br>05 January 2012 (05.01.2012),<br>claims; paragraphs [0010] to [0037], [0042] to<br>[0070]; examples<br>(Family: none) | 1-20 |

Form PCT/ISA/210 (continuation of second sheet) (July 2009)

**INTERNATIONAL SEARCH REPORT**

International application No.

PCT/JP2012/050327

Claim 19 describes as follows: "the process for producing an effervescent bath agent composition according to claim 18". However, the invention of claim 18 relates to "an effervescent bath agent composition". Therefore, the meaning of the statement of claim 19 is unclear.

Similarly, claim 20 is described as follows: "the effervescent bath agent composition according to claim 18 or 19", but this statement does not agree with the statement of claim 19.

The search was carried out with the understanding that the statement regarding to the invention described in claim 19 is a typographical error and is correctly to read "the effervescent bath agent composition according to claim 18, •••".

Form PCT/ISA/210 (extra sheet) (July 2009)

**EP 2 664 319 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 59070609 A
- JP 3230327 B
- JP 2011021005 A
- JP 011021004 A
- JP 5069805 B
- JP 2005314233 A